# EUROPEAN PATENT APPLICATION

(11) **EP 1 336 603 A1**
(43) Date of publication of application: **20.08.2003**
(21) Application number: 01978942.9
(22) Date of filing: 30.10.2001
(51) Int. Cl.: C07C 229/24, C07C 227/08, C07C 227/40, C07C 229/08

(54) **METHOD OF INTRODUCING AMINO GROUP AND METHOD OF SYNTHESIZING AMINO ACID**

(30) Priority: 17.11.2000 JP 2000350836; 05.10.2001 JP 2001309752
(71) Applicant: National Institute of Advanced Industrial Science and Technology, Tokyo 100-8921 (JP)
(72) Inventor: HATAKEDA, Kiyotaka, Sendai-shi, Miyagi 980-0874 (JP); IKUSHIMA, Yutaka, Sendai-shi, Miyagi 981-0962 (JP); SATO, Osamu, Sendai-shi, Miyagi 983-0836 (JP); KAWANAMI, Hajime, Sendai-shi, Miyagi 981-3214 (JP); KANAKUBO, Mitsuhiro, Tagajo-shi, Miyagi 985-0834 (JP); TORII, Kazuo, Sendai-shi, Miyagi 981-1105 (JP)
(74) Representative: Ebner von Eschenbach, Jennifer
(86) International application number: JP0109499
(87) International publication number: WO02040442

(57) **Abstract**

The present invention relates to a method for introducing an amino group into an organic acid by reacting the organic acid and ammonia under high-temperature and high-pressure water conditions, a method for synthesizing an amino acid by reacting an organic acid and ammonia under high-temperature and high-pressure water conditions, and thereby introducing amino group into the organic acid to synthesize an amino acid, and a method for manufacturing an amino acid by reacting an organic acid and ammonia under high-temperature and high-pressure water conditions, thereby introducing an amino group into the organic acid to synthesize an amino acid, and then separating and refining it with an ion exchange resin.

## Description

### TECHNICAL FIELD

This invention relates to, among other things, a method for introducing an amino group into an organic acid under high pressure and high temperature, and more particularly relates to a method for introducing an amino group into an organic acid by reacting an organic acid with ammonia or an ammonium salt compound under high-temperature and high-pressure water conditions, and to a method for synthesizing an amino acid from an organic acid and a method for manufacturing an amino acid from an organic acid by the above method.

The present invention makes it possible to synthesize or manufacture an amino acid under high temperature and pressure, either in batches or continuously, using an organic acid and ammonia or an ammonium salt compound as the reaction substrates, and without involving an organic solvent or catalyst in the synthesis process, and as such provides a favorable and useful method for industrial purposes.

### BACKGROUND ART

Amino acids have generally been produced in the past by many different methods such as fermentation, hydrolysis, and organic synthesis. Alanine, for instance, has been produced by microbial fermentation or by utilizing a hydrolyzate thereof, and has also been produced by organic synthesis processes in which organic reagents are used.

The following are typical examples of conventional alanine synthesis.
a) Synthesis by electrical oxidation of 3-amino-1-propanol in sulfuric acid using palladium electrodes
   (Reference: Jubilee Vol. Emil Barell, 1946, 85-91)
b) Synthesis from ethylenecyanohydrin
   (Reference: Boatright, U.S. Patent 2,734,081 (1956 to Am. Cyanamid))
c) Synthesis from β-aminopropionitrile
   (Reference: Ford, Org. Sys. Coll. Vol. III, 34 (1955))
d) Improved version of c) above
   (Reference: Beutel, Klemchuk, U.S. Patent 2,956,080 (1960 to Merck & Co.))

These and other methods that involve the use of electrode synthesis with a catalyst or ordinary synthesis have been employed.

These conventional synthesis methods require numerous factors to be taken into account, such as the disposal of used catalysts, disposal of organic solvents used in the synthesis reaction, the danger to humans posed by organic solvents, and the safe usage of these substances. Also, the larger is the scale of this synthesis, the more serious these considerations become. Therefore, there is a need for the development of technology for disposing of used organic solvents, catalysts, and so forth. On the other hand, the basic problems outlined above could be solved if some completely new synthesis method that did not involve the use of these organic solvents, catalysts, and so forth could be developed.

In light of this situation, and in view of the prior art discussed above, the inventors conducted research into a method for introducing an amino group into an organic acid under high temperature and pressure, whereupon they discovered that an amino group can be introduced into an organic acid by reacting an organic acid and ammonia or an ammonium salt compound under high-temperature and high-pressure water conditions, and perfected the present invention on the upon conducting follow-up research based on this finding.

### SUMMARY OF THE INVENTION

The present invention provides a method for introducing an amino group, a method for synthesizing an amino acid compound, and a method for manufacturing an amino acid compound, all under high-temperature and high-pressure water conditions.

The present invention is a method for introducing an amino group, wherein an amino group is introduced into an organic acid by reacting an organic acid with ammonia under high-temperature and high-pressure water conditions; a method for synthesizing an amino acid, wherein an organic acid is reacted with ammonia under high-temperature and high-pressure water conditions, and an amino group is introduced into the organic acid, thereby synthesizing an amino acid or an amino acid; and a method for manufacturing an amino acid, wherein an organic acid is reacted with ammonia under high-temperature and high-pressure water conditions, an amino group is introduced into the organic acid to synthesize an amino acid, and this product is then separated and refined with an ion exchange resin.

### DISCLOSURE OF THE INVENTION

Specifically, it is an object of the present invention to provide a novel method for introducing an amino group, in which the amino group is introduced by reacting an organic acid with ammonia or an ammonium salt compound under high-temperature and high-pressure water conditions.

It is a further object of the present invention to provide a novel method for synthesizing an amino acid, in which an amino acid is synthesized by the above-mentioned amino group introduction method from an organic acid and ammonia or an ammonium salt compound.

It is a further object of the present invention to provide a novel method for synthesizing an amino acid, in which an amino acid is synthesized from an organic acid by the above-mentioned amino group introduction method, such as synthesizing alanine from a hydroxy acid type of lactic acid and ammonia or an ammonium salt, glycine from glycolic acid, aspartic acid from malic acid, or α,β-diaminosuccinic acid from tartaric acid.

It is a further object of the present invention to provide a method for synthesizing an amino acid compound by batch process, in which an organic acid and ammonia or an ammonium salt compound are introduced into a reactor under high-temperature and high-pressure water conditions, or a continuous method for synthesizing an amino acid, in which an amino acid is continuously synthesized.

It is a further object of the present invention to provide a method for manufacturing a high-purity amino acid, wherein an amino acid is synthesized from an organic acid and ammonia or an ammonium salt compound by the above-mentioned amino group introduction method, and the amino acid is separated and refined from the resulting reaction solution by using an ion exchange resin or another such amino acid separation material.

To solve the above problems, the present invention is constituted by the following technological means.
(1) A method for introducing an amino group, wherein an amino group is introduced into an organic acid by reacting the organic acid with ammonia or an ammonium salt compound under high-temperature and high-pressure water conditions.
(2) A method for synthesizing an amino acid, wherein an organic acid is reacted with ammonia or an ammonium salt compound under high-temperature and high-pressure water conditions, and an amino group is introduced into the organic acid to synthesize an amino acid.
(3) The method for synthesizing an amino acid according to (2) above, wherein an organic acid is reacted with ammonia or an ammonium salt compound under high-temperature and high-pressure water conditions in which the temperature is at least 250°C and the pressure is at least 20 MPa.
(4) The method for synthesizing an amino acid according to (2) or (3) above, wherein an organic acid is reacted with aqueous ammonia, ammonium acetate, or ammonium carbonate under high-temperature and high-pressure water conditions.
(5) The method for synthesizing an amino acid according to (2) or (3) above, wherein an organic acid is reacted with liquid ammonia under high-temperature and high-pressure water conditions.
(6) The method for synthesizing an amino acid according to any of (2) to (5) above, wherein a hydroxy acid is used as the organic acid.
(7) The method for synthesizing an amino acid according to any of (2) to (6) above, wherein lactic acid, glycolic acid, malic acid, or tartaric acid is used as the organic acid.
(8) The method for synthesizing an amino acid according to any of (2) to (7) above, wherein an organic acid and ammonia or an ammonium salt compound are introduced into a reactor and continuously reacted under high-temperature and high-pressure water conditions.
(9) A method for manufacturing an amino acid using an organic acid and ammonia or an ammonium salt compound as the reaction substrates under high-temperature and high-pressure water conditions, wherein the organic acid and the ammonia or ammonium salt compound are introduced into a reactor and continuously reacted under high-temperature and high-pressure water conditions, and after the reaction the obtained reaction solution is separated and refined with an ion exchange resin or another such amino acid separation material to obtain an amino acid.

The present invention will now be described in further detail.

To facilitate an understanding of the present invention, the following detailed description is of a case in which ammonia or an ammonium salt compound is reacted under high temperature and pressure with a corresponding hydroxy acid to synthesize alanine, glycine, aspartic acid, or α,β-diaminosuccinic acid, which are amino acids, but the present invention is not limited to or by these examples.

A typical example of the synthesis method of the present invention, which the inventors developed through various experiments, is a method in which alanine is synthesized by introducing lactic acid and an ammonia aqueous solution, an ammonium carbonate aqueous solution, or an ammonium acetate aqueous solution into a reactor under high-temperature and high-pressure water conditions, and passing these compounds through the reactor at high speed. The raw material reagents used in the synthesis method of the present invention are just an organic acid and ammonia or an ammonium salt compound. Water under high temperature and high pressure is used as a reaction solvent or reaction site in the present invention, and no organic solvent or catalyst is used, nor is there any particular need to use these. Therefore, when this method is used, there is no discharge of wastes that would have to be disposed of, such as waste solvent or spent catalyst. Also, any unreacted organic acid or used water can be reused in the reaction of the present invention. Further, since the method of the present invention allows useful amino acids and other such products to be synthesized continuously and at high speed, it is believed to be an ideal method for manufacturing these products. This reaction can also be carried out in a batch reactor.

The amino group introduction method and amino acid synthesis method of the present invention will now be described in detail.

With the present invention, an amino acid can be synthesized, for example, by reacting an α-hydroxy acid and ammonia or an ammonium salt compound under high-temperature and high-pressure water conditions, and introducing an amino group into the α-hydroxy acid.

In general, amino acids are produced mainly in vivo by living organisms, but can also be manufactured by organic synthesis. In the case of alanine, for instance, β-alanine can be synthesized by reacting β-propiolactone with ammonia in an acetonitrile solvent.

### Chemical Formula 1

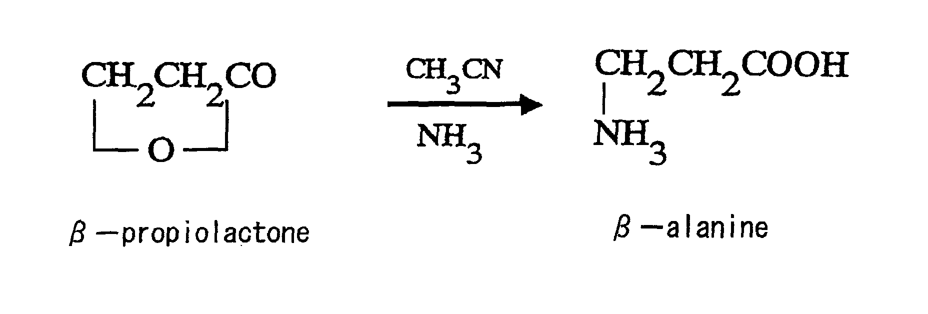

In contrast, the inventors discovered that alanine, glycine, aspartic acid, α,β-diaminosuccinic acid, and other such amino acids can be synthesized by reacting an organic acid such as lactic acid, glycolic acid, malic acid, tartaric acid, and the like which are α-hydroxy acids, respectively, with ammonia or an ammonium salt compound under high-temperature and high-pressure water conditions.

The synthesis reaction formulas for alanine, glycine, aspartic acid, and α,β-diaminosuccinic acid are given below as specific examples of the amino acid synthesis pertaining to the present invention.

### Chemical Formula 2

### Reaction formula for alanine synthesis

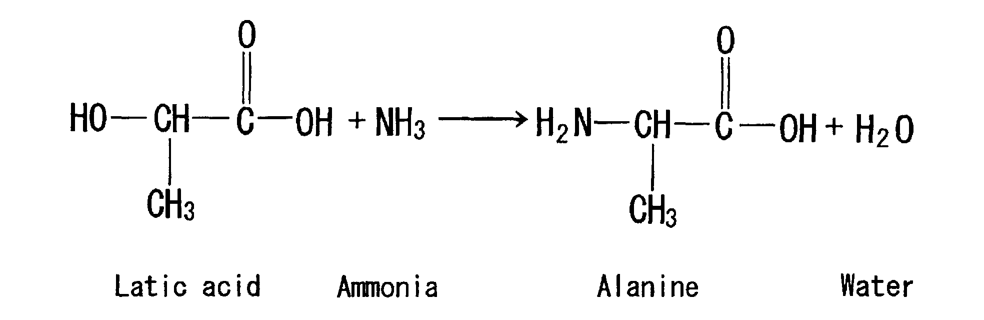

### Chemical Formula 3

### Reaction formula for glycine synthesis

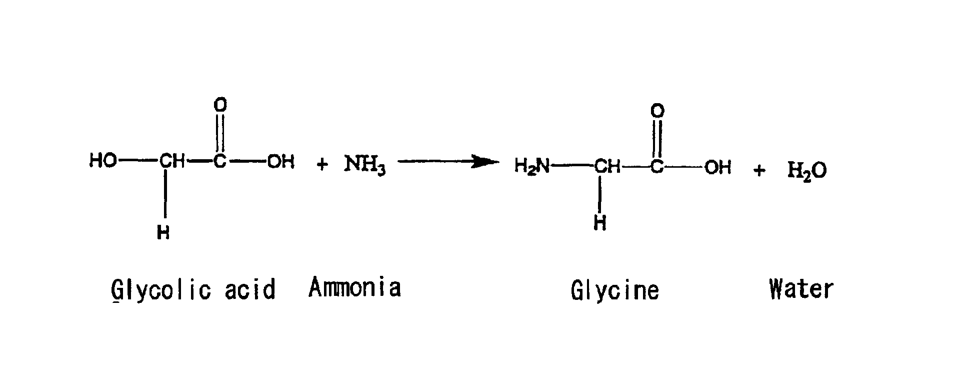

### Chemical Formula 4

### Reaction formula for aspartic acid synthesis

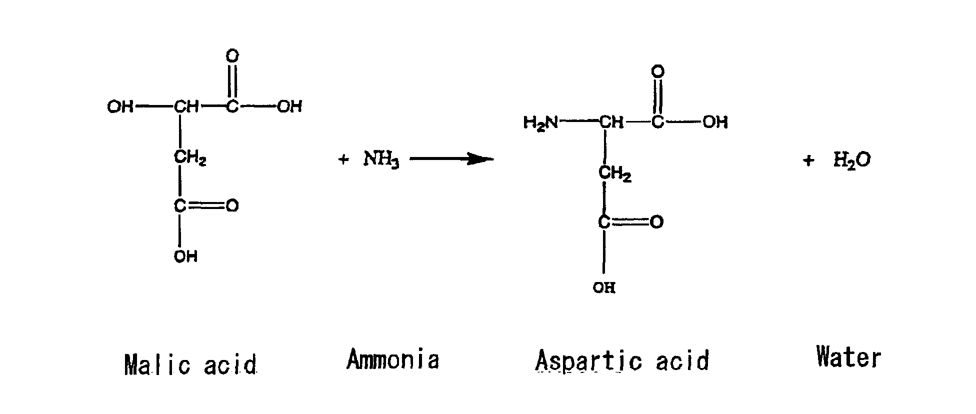

### Chemical Formula 5

### Reaction formula for α,β-diaminosuccinic acid synthesis

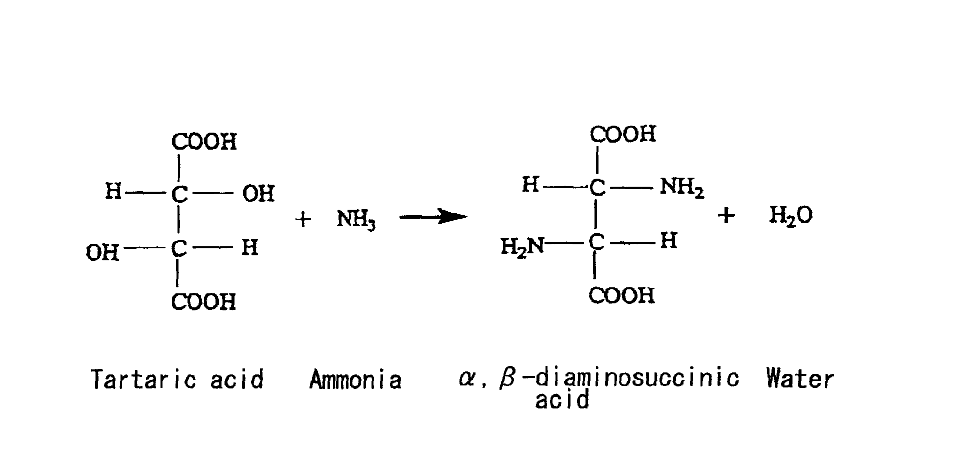

The temperature of the high-temperature and high-pressure water in the method of the present invention can be controlled from outside the reactor by using a heater, a molten salt, or the like. The temperature can also be controlled by an endothermic approach within the reactor. High-temperature and high-pressure water can also be manufactured ahead of time and injected into the reactor from the outside. It is also possible to control the reaction conditions by supplying two or more types of high-temperature and high-pressure water under different temperature and pressure conditions to the reactor. The pressure inside the reaction vessel can be controlled with a pressure regulator valve in a flow system. As to the reaction pressure in a batch process, the spontaneous pressure at the usage temperature can be calculated, for example. Furthermore, the pressure can be controlled by injecting nitrogen gas or another vapor. It is generally best for the pressure to be no lower than the spontaneous pressure at the usage temperature.

Basically, the present invention is achieved under high-temperature and high-pressure water conditions in which the temperature is at least 250°C and the pressure is at least 20 MPa, but the present invention can be achieved even more preferably under high-temperature and high-pressure water conditions in which the temperature is at least 300°C and the pressure is at least 25 MPa. Furthermore, the present invention will be achieved most favorably if the high-temperature and high-pressure water conditions are selected so that the temperature is between 300 and 420°C and the pressure is between 25 and 50 MPa. The ideal temperature conditions will vary with the treatment time, but can generally be selected favorably from a temperature range of 250 to 450°C. The suitable temperature and pressure conditions can be selected as dictated by the treatment quantity and the reaction apparatus.

A high-temperature and high-pressure reaction apparatus can be used, for example, but the type of reaction apparatus is not limited, as long as it is one that allows the reaction conditions to be set to high-temperature and high-pressure water conditions.

Examples of a favorable reaction apparatus include the batch reaction apparatus and the high-temperature and high-pressure reaction apparatus of flow system used in the present invention. A commercially available autoclave can be used favorably.

The reaction conditions in the method of the present invention will vary with the type and concentration of the organic acid being used, the type and concentration of the ammonia or ammonium salt compound, the reaction time, and the high-temperature and high-pressure water conditions.

With the present invention, examples of the organic acid serving as the reaction substrate include lactic acid, glycolic acid, malic acid, tartaric acid, hydroxy-n-butyric acid, mandelic acid, 2-hydroxy-3-methylbutyl acid, citric acid, glyceric acid, tropic acid, benzylic acid, hydroxyvaleric acid, and other such hydroxy acids having at least one carboxyl group and at least one hydroxyl group per molecule. As hydroxy acids, α-hydroxy acids, β-hydroxy acids, γ-hydroxy acids, δ-hydroxy acids, ε-hydroxy acids, and so forth can all be used favorably in the reaction. The organic acid used in the reaction of the present invention is not limited to a single type, and the reaction will also proceed favorably when a mixture of two or more types is used. Hydroxycarboxylic acids such as aliphatic saturated hydroxycarboxylic acids, aliphatic unsaturated hydroxycarboxylic acids, or aromatic hydroxycarboxylic acids, steroids, and other such organic acids can all be used favorably as the reaction substrate in the present invention.

Metal salts of the above-mentioned organic acids can also be used as the raw material in the present invention. For example, sodium, potassium, magnesium, calcium, ammonium, and other such organic salts can be used favorably.

When an apparatus of flow system is used, the concentration of the organic acid to be introduced into the reactor can be controlled, for example, by controlling the flux of the high-temperature and high-pressure water used as the carrier water or the introduction flux of the organic acid serving as the reaction substrate. The organic acid and the ammonia or ammonium salt compound can be supplied simultaneously or individually to the reaction after being dissolved in carrier water. The concentration of the organic acid to be introduced into the reactor can usually be selected from a range of 1 mM to 10 M. Preferably, a suitable concentration thereof is selected from a range of 2 mM to 5 M, and ideally a suitable concentration thereof is selected from a range of 4 mM to 2 M, but the present invention is not limited to these concentration values. With a batch process, the concentration of the organic acid to be supplied just needs to be controlled. The concentration of the organic acid within the reactor will vary with the density of the high-temperature and high-pressure water participating in the reaction.

With the present invention, the quantity of amino groups to be introduced, the position where the amino groups are introduced, the type of amino acid to be produced, the production amount, and the reaction yield can be manipulated by adjusting the temperature and pressure of the reaction system, the reaction time, the concentration of the reaction substrate, and the concentration of the ammonia or ammonium salt, according to the type of organic acid in question.

Liquefied ammonia or aqueous ammonia with a concentration of 28% can usually be used favorably as the ammonia to be used in the reaction, but the reaction will still proceed if gaseous ammonia is introduced into the high-temperature and high-pressure water. Ammonium salt compounds that can be used favorably include ammonium acetate, ammonium carbonate, ammonium formate, ammonium chloride, and ammonium sulfate.

In most cases, the ammonia or ammonium salt compound is introduced into the reactor after being mixed with the organic salt or organic ester serving as the reaction substrate. Here, the ammonia or ammonium salt compound is usually used in the form of an aqueous solution, and ultimately becomes an aqueous solution even when liquefied ammonia is used, and the reaction concentration can be suitably selected from a range of 1 to 10 times the organic salt or organic ester concentration. For instance, the concentration of an ammonia aqueous solution or ammonium aqueous solution can be selected from a range of 1 mM to 20 M, and preferably 2 mM to 10 M. The best range is from 4 mM to 8 M, but in the present invention the concentration is not limited to these concentration values. The reaction of the present invention will proceed whether the organic acid and the ammonia or ammonium salt compound are introduced separately into the reactor, or are used after being directly mixed in carrier water. The reaction of the present invention can also be achieved by using a mixture of ammonia and an ammonium salt.

In the reaction system of the present invention, an organic acid and ammonia or an ammonium salt compound serving as the reaction substrates should be present in high-temperature and high-pressure water with a temperature of at least 250°C and a pressure of at least 20 MPa, but there is no particular need to add a water-soluble catalyst such as metal ions, an acid, or a base, or a metal-supported catalyst, a solid acid, a solid base, or another such solid catalyst, or an enzyme, or the like, nor is there any need to use an organic solvent.

The present invention is basically characterized in that the above-mentioned reaction substrates are made to be present in high-temperature and high-pressure water, and the organic acid and the ammonia or ammonium salt compound are reacted in the absence of a catalyst, or without the participation of an organic solvent in the reaction, so that an amino group is introduced into the organic acid, and in that if needed, methanol, ethanol, ethylene glycol, or another such organic solvent, metal ions, an acid, a base, or another such water-soluble catalyst, or a metal-supported catalyst, a solid acid, a solid base, or another such solid catalyst, or an enzyme may be participated in the reaction.

With the present invention, the above-mentioned reaction system results in an amino group being introduced into the organic acid, or an amino acid being synthesized, in a very short reaction time of about 0.001 second to 10 minutes. For example, when a reaction apparatus of flow system is used, the reaction time can be controlled by controlling the reaction temperature, reaction pressure, flow rate of the high-temperature and high-pressure water, introduction rate of the reaction substrate, size of the reactor, length of the flow passages in the reactor, and other such factors. Preferably, the reaction time is selected from a range of 0.01 second to 5 minutes, with the best range being from 0.05 second to 2 minutes, but the present invention is not limited to these values.

As shown in the examples given below, the inventors have used a high performance liquid chromatography/mass spectroscopy apparatus (LC-MS apparatus) and a Fourier infrared spectrophotometer (FTIR apparatus) to confirm that an amino group can be introduced into an organic acid in a short time (such as a reaction time of about 0.1 second) under high-temperature and high-pressure water conditions. Furthermore, they used an LC-MS apparatus to isolate and identify the types of organic acid and amino acid, and to accurately quantify the amounts thereof contained. They also used an ion exchange resin and the like to separate and refine a continuously obtained amino acid, measured the infrared absorption spectrum with an FTIR apparatus, and compared this spectrum with that of a guaranteed reagent of high purity, which afforded accurate identification of the type of amino acid. Similarly, the type and purity of an amino acid can be confirmed by NMR measurement.

For example, aspartic acid with a concentration of 0.2 to 7.3 mM was synthesized from aqueous ammonia and malic acid with a concentration of 4.8 mM to 47.8 mM using an apparatus of flow system at 300 to 420°C, a pressure of 35 MPa, and a reaction time of 0.06 to 0.33 second. Similarly, in a batch process, alanine with a concentration of 13.7 mM was synthesized from aqueous ammonia and lactic acid at a temperature of 350°C, a pressure of 30 MPa, and a reaction time of 40 seconds. As a result of these reactions, the ammonia reacted with the hydroxyl groups in the organic acid, the hydroxyl groups were removed, and amino groups were introduced in place, which was confirmed with an LC-MS apparatus, an NMR measurement apparatus, and an FTIR apparatus.

The reaction yield of the amino acid produced with the present invention varies with the temperature, pressure, and other reaction conditions, the type of organic acid, the concentration of the organic acid, the concentration of the ammonia or ammonium compound, the configuration of the reaction apparatus, the size of the reactor, and other such factors. For instance, the reaction yield was from 4.2% to 21.3% when aspartic acid was synthesized using an apparatus of flow system. This aspartic acid was recovered as a mixture with the raw material malic acid and so forth. Similarly, many types of amino acid are recovered along with the raw material substrates from various organic acids or mixtures thereof, but the amino acid can be separated and refined from the raw material substrates by treating the reaction solution obtained after the reaction with an ion exchange resin, such as a cation exchange resin, an anion exchange resin, or a mixture of these. Furthermore, since the amino acids can be separated from one another, each type of amino acid can be refined and concentrated. Also, the raw material substrates recovered at the same time can be reused as raw materials. Suitable ordinary amino acid separating materials, such as alumina, silica gel for reversed phase, zeolite, cellulose, and carbon, can also be utilized instead of an ion exchange resin to separate and refine the amino acid.

Therefore, a high-purity amino acid can be favorably manufactured by reacting an organic acid and ammonia or an ammonium salt compound under high-temperature and high-pressure water conditions to synthesize an amino acid, and then refining out the amino acid from the obtained reaction solution using an ion exchange resin, alumina, silica gel for reversed phase, cellulose, or another such material for separating amino acids.

With the present invention, having an organic acid and ammonia or an ammonium salt compound be present in a specific concentration as reaction substrates in hot water under high-temperature and high-pressure water conditions allows, for example, alanine to be synthesized from an α-hydroxy acid type of lactic acid and ammonia. In this case, if glycolic acid, malic acid, or tartaric acid is reacted instead of lactic acid with ammonia, an amino group will be introduced into this organic acid, synthesizing glycine, aspartic acid, or α,β-diaminosuccinic acid. Also, continuously introducing one of these organic acids or the like and an ammonia aqueous solution or an ammonium salt compound into the reactor allows various types of amino acids corresponding to the respective organic acids to be synthesized continuously.

The above makes it clear that with the present invention, an amino group can be introduced into an organic acid by adjusting the reaction conditions in the above-mentioned reaction system, the type of organic acid serving as the reaction substrate, and the concentration of the organic acid and the ammonia aqueous solution or ammonium salt compound, and that this allows an amino acid to be synthesized in a short time, which means that the present invention is useful as a novel method for introducing amino groups, method for synthesizing amino acids, and method for manufacturing amino acids.

### BRIEF DESCRIPTION OF THE DRAWINGS

Fig. 1 is a flow sheet for a reaction apparatus of flow system equipped with two water pumps to be used in the present invention;

Fig. 2 is a flow sheet for a reaction apparatus of flow system equipped with three water pumps to be used in the present invention; and

Fig. 3 shows the main components of a salt bath having means of shaking and agitating using a batch reaction tube and mixed salts of a sodium nitrate/potassium nitrate, which is used in a bath reaction.

### BEST MODE FOR CARRYING OUT THE INVENTION

The present invention will now be described in specific terms through examples, but the present invention is not limited in any way by the following examples.

### Example 1

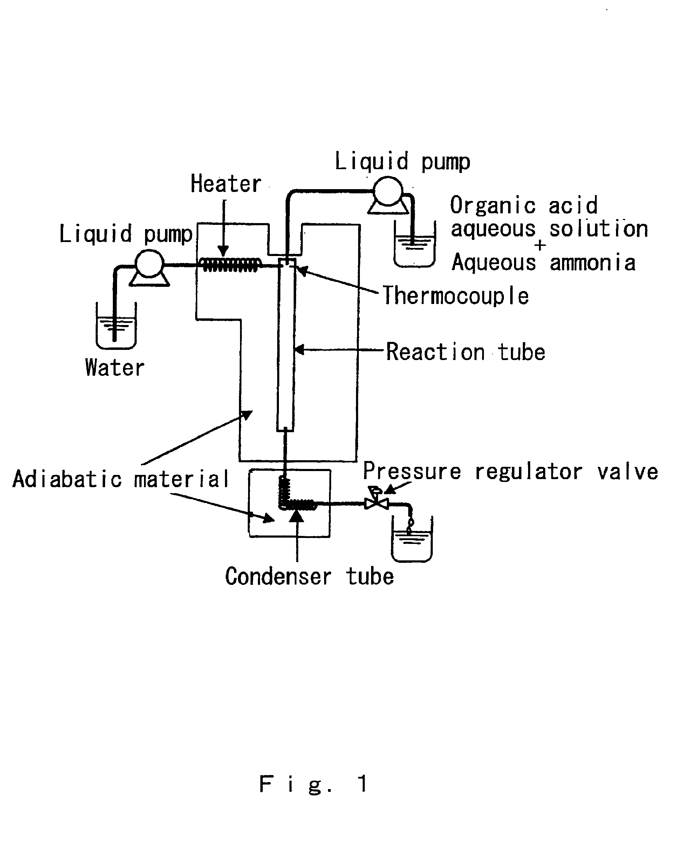

Using the continuous reaction apparatus shown in Fig. 1, malic acid (reagent chemicals made by Wako Pure Chemicals) and aqueous ammonia (reagent chemicals made by Wako Pure Chemicals) were reacted under high-temperature and high-pressure water conditions in which the temperature was 350°C, the pressure was 35 MPa, and the density was 0.6593 g/cm³, to attempt the continuous synthesis of aspartic acid by the introduction of amino groups.

The reactor was made from alloy C-276, the inside diameter of the reactor was 0.65 mm, and the reactor length was 25 cm. The reactor volume was therefore calculated to be 0.083 cm³. A high-pressure injection pump was used to introduce the various preparations. The water used in the reaction was distilled water, and carrier water from which dissolved oxygen had been purged by bubbling with nitrogen gas was sent through the system at a rate of 9 mL/min. Using similarly treated distilled water, a substrate solution containing 0.100 M malic acid and 0.300 M aqueous ammonia was prepared, and this substrate solution was introduced into the reactor at a flow rate of 4.7 mL/min. The concentrations of the substrates prior to entering the reactor were 34.3 mM for malic acid and 0.1028 M for aqueous ammonia, and the reaction time was 0.240 second. The aqueous solution obtained after the reaction was examined with a high performance liquid chromatography/mass spectroscopy apparatus, which confirmed that an amino group had been introduced into the malic acid, and that aspartic acid had been produced. The concentration of the aspartic acid contained was 7.3 mM, and the reaction yield thereof was 21.3%.

### Example 2

Malic acid and aqueous ammonia were continuously reacted for one hour under exactly the same conditions as in Example 1. The reaction solution thus obtained was passed through a cation exchange resin (50W-X8, made by Dow Chemical) column to separate the produced aspartic acid from the raw material malic acid, and the aspartic acid-containing solution was concentrated and refined, after which it was precipitated with ethanol, and the precipitate was filtered and dried to obtain 0.78 g of the product of the present invention. This product was in the form of a pure-white powder, and was confirmed from FTIR absorption spectrum measurement results and NMR measurement results to be high-purity aspartic acid containing substantially no impurities.

### Example 3

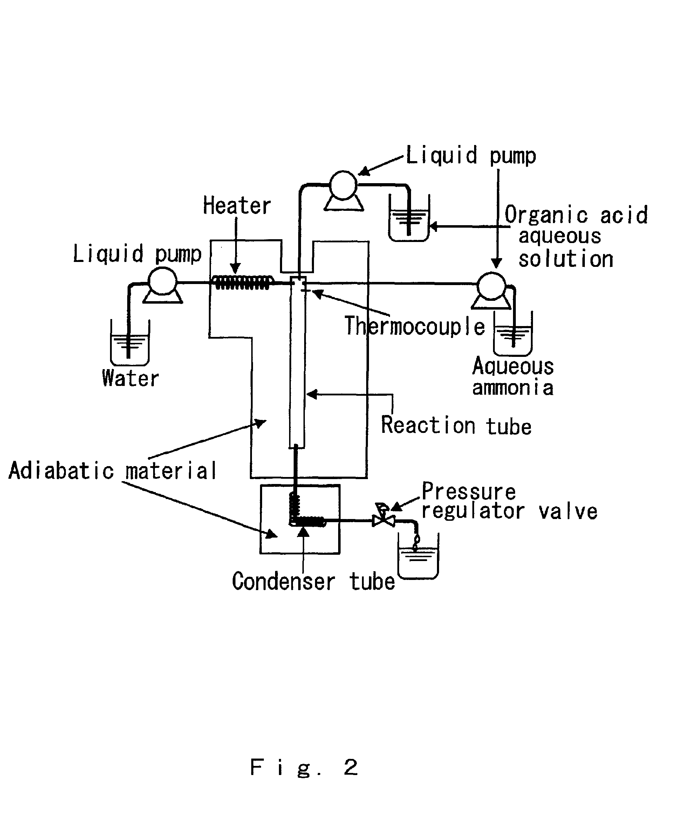

The continuous synthesis of aspartic acid from malic acid and aqueous ammonia was attempted by conducting the same reaction as in Example 1. Also, using the continuous reaction apparatus shown in Fig. 2, a 0.100 M malic acid aqueous solution and a 0.300 M ammonia aqueous solution that had each been separately prepared were injected into the reactor by two different water pumps. Some of the reaction conditions, however, were changed as follows.

| Changed reaction conditions | |
|---|---|
| Reaction temperature | 374°C |
| Density of high-temperature and high-pressure water | 0.588 g/cm³ |
| Flow rate of carrier water | 10 mL/min |
| Flow rate of substrate solution (0.100 M malic acid aqueous solution) | 3.2 mL/min |
| Flow rate of substrate solution (0.300 M ammonia aqueous solution) | 3.2 mL/min |

The concentrations of the substrates prior to entering the reactor were 19.5 mM for malic acid and 0.0585 M for aqueous ammonia. The reaction time was 0.222 second, and the aqueous solution obtained after the reaction was examined with a high performance liquid chromatography/mass spectroscopy apparatus, whereby it was confirmed that an amino group had been introduced into the malic acid, and that aspartic acid had been produced. The concentration of the aspartic acid was 4.6 mM, and the reaction yield thereof was 23.6%.

### Example 4

The continuous synthesis of aspartic acid from malic acid and aqueous ammonia was attempted by conducting the same reaction as in Example 1. Some of the reaction conditions, however, were changed as follows.

| Changed reaction conditions | |
|---|---|
| Reaction temperature | 300°C |
| Density of high-temperature and high-pressure water | 0.7580 g/cm³ |
| Flow rate of carrier water | 6 mL/min |
| Flow rate of substrate solution | 5.5 mL/min |

The concentrations of the substrates prior to entering the reactor were 47.8 mM for malic acid and 0.1434 M for aqueous ammonia. The reaction time was 0.328 second, and the aqueous solution obtained after the reaction was examined with a high performance liquid chromatography/mass spectroscopy apparatus, whereby it was confirmed that an amino group had been introduced into the malic acid, and that aspartic acid had been produced. The concentration of the aspartic acid was 5.4 mM, and the reaction yield thereof was 11.3%.

### Comparative Example

The continuous synthesis of aspartic acid from malic acid and aqueous ammonia was attempted by conducting the same reaction as in Example 1. Some of the reaction conditions, however, were changed as follows.

| Changed reaction conditions | |
|---|---|
| Reaction temperature | 200°C |
| Reaction pressure | 15 MPa |
| Density of high-temperature and high-pressure water | 0.8746 g/cm³ |
| Flow rate of carrier water | 6 mL/min |
| Flow rate of substrate solution | 5.5 mL/min |

The concentrations of the substrates prior to entering the reactor were 47.8 mM for malic acid and 0.1434 M for aqueous ammonia. The reaction time was 0.379 second, and the aqueous solution obtained after the reaction was examined with a high performance liquid chromatography/mass spectroscopy apparatus, whereby it was revealed that only the raw material malic acid could be detected, and no aspartic acid whatsoever had been obtained.

### Example 5

The continuous synthesis of aspartic acid from malic acid and aqueous ammonia was attempted by conducting the same reaction as in Example 1. Some of the reaction conditions, however, were changed as follows.

| Changed reaction conditions | |
|---|---|
| Reaction temperature | 420°C |
| Density of high-temperature and high-pressure water | 0.2700 g/cm³ |
| Flow rate of carrier water | 20 mL/min |
| Flow rate of substrate solution | 1 mL/min |

The concentrations of the substrates prior to entering the reactor were 4.8 mM for malic acid and 0.0142 M for aqueous ammonia. The reaction time was 0.064 second, and the aqueous solution obtained after the reaction was examined with a high performance liquid chromatography/mass spectroscopy apparatus, whereby it was confirmed that an amino group had been introduced into the malic acid, and that aspartic acid had been produced. The concentration of the aspartic acid was 0.2 mM, and the reaction yield thereof was 4.2%.

### Example 6

The continuous synthesis of glycine from glycolic acid and aqueous ammonia (reagent chemicals made by Wako Pure Chemicals) was attempted by conducting the same reaction as in Example 1. A substrate solution containing 0.100 M glycolic acid and 0.199 M aqueous ammonia, which was prepared using distilled water from which dissolved oxygen had been removed, was supplied to the reaction. Some of the reaction conditions, however, were changed as follows.

| Changed reaction conditions | |
|---|---|
| Reaction temperature | 374°C |
| Density of high-temperature and high-pressure water | 0.588 g/cm³ |
| Flow rate of carrier water | 6 mL/min |
| Flow rate of substrate solution | 2.3 mL/min |

The concentrations of the substrates prior to entering the reactor were 27.7 mM for glycolic acid and 55.2 mM for aqueous ammonia. The reaction time was 0.353 second, and the aqueous solution obtained after the reaction was examined with a high performance liquid chromatography/mass spectroscopy apparatus, whereby it was confirmed that an amino group had been introduced into the glycolic acid, and that glycine had been produced. The glycine concentration was 1.2 mM, and the reaction yield thereof was 4.3%.

### Example 7

The continuous synthesis of glycine from glycolic acid and aqueous ammonia was attempted by conducting the same reaction as in Example 6. Some of the reaction conditions, however, were changed as follows.

| Changed reaction conditions | |
|---|---|
| Reaction temperature | 400°C |
| Density of high-temperature and high-pressure water | 0.4749 g/cm³ |
| Flow rate of carrier water | 10 mL/min |
| Flow rate of substrate solution | 2 mL/min |

The concentrations of the substrates prior to entering the reactor were 16.7 mM for glycolic acid and 33.2 mM for aqueous ammonia. The reaction time was 0.197 second, and the aqueous solution obtained after the reaction was examined with a high performance liquid chromatography/mass spectroscopy apparatus, whereby it was confirmed that an amino group had been introduced into the glycolic acid, and that glycine had been produced. The glycine concentration was 0.7 mM, and the reaction yield thereof was 4.2%.

### Example 8

The continuous synthesis of alanine from lactic acid (reagent chemicals made by Wako Pure Chemicals) and aqueous ammonia was attempted by conducting the same reaction as in Example 1. A substrate solution containing 1.085 M lactic acid and 5.002 M aqueous ammonia, which was prepared using distilled water from which dissolved oxygen had been removed, was supplied to the reaction. Some of the reaction conditions, however, were changed as follows.

| Changed reaction conditions | |
|---|---|
| Reaction temperature | 374°C |
| Density of high-temperature and high-pressure water | 0.588 g/cm³ |
| Flow rate of carrier water | 5 mL/min |
| Flow rate of substrate solution | 2 mL/min |

The concentrations of the substrates prior to entering the reactor were 0.310 M for lactic acid and 1.429 M for aqueous ammonia. The reaction time was 0.418 second, and the aqueous solution obtained after the reaction was examined with a high performance liquid chromatography/mass spectroscopy apparatus, whereby it was confirmed that an amino group had been introduced into the lactic acid, and that alanine had been produced. The alanine concentration was 8.6 mM, and the reaction yield thereof was 2.8%.

### Example 9

The continuous synthesis of alanine from lactic acid and aqueous ammonia was attempted by conducting the same reaction as in Example 8. Some of the reaction conditions, however, were changed as follows.

| Changed reaction conditions | |
|---|---|
| Reaction pressure | 40 MPa |
| Density of high-temperature and high-pressure water | 0.6090 g/cm³ |
| Flow rate of carrier water | 4.5 mL/min |
| Flow rate of substrate solution | 1.35 mL/min |

The concentrations of the substrates prior to entering the reactor were 0.250 M for lactic acid and 1.154 M for aqueous ammonia. The reaction time was 0.518 second, and the aqueous solution obtained after the reaction was examined with a high performance liquid chromatography/mass spectroscopy apparatus, whereby it was confirmed that an amino group had been introduced into the lactic acid, and that alanine had been produced. The alanine concentration was 7.7 mM, and the reaction yield thereof was 3.1%.

### Example 10

The continuous synthesis of alanine from lactic acid and aqueous ammonia was attempted by conducting the same reaction as in Example 8. Some of the reaction conditions, however, were changed as follows.

| Changed reaction conditions | |
|---|---|
| Reaction pressure | 30 MPa |
| Density of high-temperature and high-pressure water | 0.5580 g/cm³ |
| Flow rate of carrier water | 9.3 mL/min |
| Flow rate of substrate solution | 4.5 mL/min |

The concentrations of the substrates prior to entering the reactor were 0.354 M for lactic acid and 1.631 M for aqueous ammonia. The reaction time was 0.201 second, and the aqueous solution obtained after the reaction was examined with a high performance liquid chromatography/mass spectroscopy apparatus, whereby it was confirmed that an amino group had been introduced into the lactic acid, and that alanine had been produced. The alanine concentration was 7.3 mM, and the reaction yield thereof was 2.1%.

### Example 11

The continuous synthesis of alanine from lactic acid and aqueous ammonia was attempted by conducting the same reaction as in Example 8. Some of the reaction conditions, however, were changed as follows.

| Changed reaction conditions | |
|---|---|
| Reaction pressure | 25 MPa |
| Density of high-temperature and high-pressure water | 0.4851 g/cm³ |
| Flow rate of carrier water | 6.7 mL/min |
| Flow rate of substrate solution | 2 mL/min |

The concentrations of the substrates prior to entering the reactor were 0.249 M for sodium glycolate and 1.150 M for aqueous ammonia. The reaction time was 0.278 second, and the aqueous solution obtained after the reaction was examined with a high performance liquid chromatography/mass spectroscopy apparatus, whereby it was confirmed that an amino group had been introduced into the lactic acid, and that alanine had been produced. The alanine concentration was 4.5 mM, and the reaction yield thereof was 1.8%.

### Example 12

The continuous synthesis of alanine from lactic acid and aqueous ammonia was attempted by conducting the same reaction as in Example 8. Some of the reaction conditions, however, were changed as follows.

| Changed reaction conditions | |
|---|---|
| Reaction temperature | 400°C |
| Reaction pressure | 40 MPa |
| Density of high-temperature and high-pressure water | 0.5237 g/cm³ |
| Flow rate of carrier water | 6 mL/min |
| Flow rate of substrate solution | 1 mL/min |

The concentrations of the substrates prior to entering the reactor were 0.155 M for lactic acid and 0.715 M for aqueous ammonia. The reaction time was 0.373 second, and the aqueous solution obtained after the reaction was examined with a high performance liquid chromatography/mass spectroscopy apparatus, whereby it was confirmed that an amino group had been introduced into the lactic acid, and that alanine had been produced. The alanine concentration was 5.5 mM, and the reaction yield thereof was 3.6%.

### Example 13

The continuous synthesis of alanine from lactic acid and ammonium acetate (reagent chemicals made by Wako Pure Chemicals) was attempted by conducting the same reaction as in Example 1. A substrate solution containing 1.085 M lactic acid and 1.409 M ammonium acetate, which was prepared using distilled water from which dissolved oxygen had been removed, was supplied to the reaction. Some of the reaction conditions, however, were changed as follows.

| Changed reaction conditions | |
|---|---|
| Reaction temperature | 374°C |
| Density of high-temperature and high-pressure water | 0.588 g/cm³ |
| Flow rate of carrier water | 7 mL/min |
| Flow rate of substrate solution | 3 mL/min |

The concentrations of the substrates prior to entering the reactor were 0.325 M for lactic acid and 0.423 M for the ammonium acetate aqueous solution. The reaction time was 0.293 second, and the aqueous solution obtained after the reaction was examined with a high performance liquid chromatography/mass spectroscopy apparatus, whereby it was confirmed that an amino group had been introduced into the lactic acid, and that alanine had been produced. The alanine concentration was 6.0 mM, and the reaction yield thereof was 1.8%.

### Example 14

The continuous synthesis of alanine from lactic acid and ammonium carbonate (reagent chemicals made by Kokusan Chemical) was attempted by conducting the same reaction as in Example 1. A 1.153 M ammonium carbonate aqueous solution, which was prepared using distilled water from which dissolved oxygen had been removed, was used as the carrier water. A 1.084 M lactic acid substrate solution, which was prepared using distilled water from which dissolved oxygen had been removed, was supplied to the reaction. Some of the reaction conditions, however, were changed as follows.

| Changed reaction conditions | |
|---|---|
| Reaction temperature | 374°C |
| Reaction pressure | 30 MPa |
| Density of high-temperature and high-pressure water | 0.558 g/cm³ |
| Flow rate of carrier water | 7 mL/min |
| Flow rate of substrate solution | 3 mL/min |

The concentrations of the substrates prior to entering the reactor were 0.3252 M for lactic acid and 0.8071 M for the ammonium carbonate aqueous solution. The reaction time was 0.278 second, and the aqueous solution obtained after the reaction was examined with a high performance liquid chromatography/mass spectroscopy apparatus, whereby it was confirmed that an amino group had been introduced into the lactic acid, and that alanine had been produced. The alanine concentration was 6.5 mM, and the reaction yield thereof was 2.0%.

### Example 15

The continuous synthesis of α,β-diaminosuccinic acid from L-tartaric acid (reagent chemicals made by Nakarai Chemical) and aqueous ammonia was attempted by conducting the same reaction as in Example 1. A substrate solution containing 1.00 M L-tartaric acid and 4.61 M aqueous ammonia, which was prepared using distilled water from which dissolved oxygen had been removed, was supplied to the reaction. Some of the reaction conditions, however, were changed as follows.

| Changed reaction conditions | |
|---|---|
| Reaction temperature | 374°C |
| Density of high-temperature and high-pressure water | 0.588 g/cm³ |
| Flow rate of carrier water | 10 mL/min |
| Flow rate of substrate solution | 4.5 mL/min |

The concentrations of the substrates prior to entering the reactor were 0.3103 M for tartaric acid and 1.4307 M for aqueous ammonia. The reaction time was 0.202 second, and the aqueous solution obtained after the reaction was examined with a high performance liquid chromatography/mass spectroscopy apparatus, whereby it was confirmed that two amino groups had been introduced into the tartaric acid, and that α,β-diaminosuccinic acid had been produced. The α,β-diaminosuccinic acid concentration was 0.75 mM, and the reaction yield thereof was 0.2%.

### Example 16

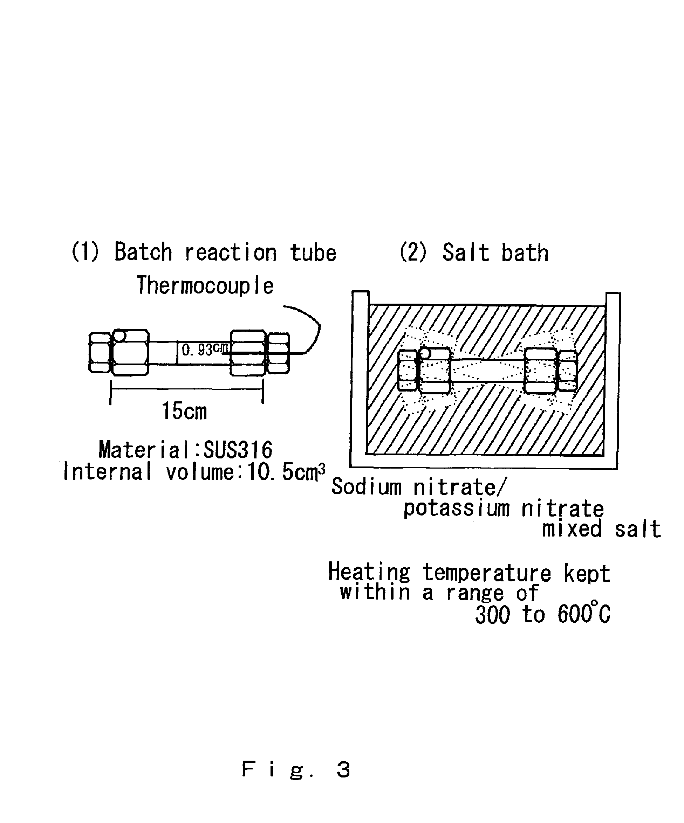

The introduction of an amino group into lactic acid was attempted under high-temperature and high-pressure water conditions using lactic acid and aqueous ammonia as the reaction substrates. The reaction was conducted in a batch reaction apparatus capable of shaking and agitating the reaction solution, as shown in Fig. 3. A reaction tube with an internal volume of 10.5 cm³ was used as the reactor, and the reaction tube was put into a sodium nitrate/potassium nitrate mixed salt bath, the temperature thereof was set to 350°C and the pressure to 30 Mpa, for 60 seconds to conduct an amino group introduction reaction. It took 40 seconds for the system to rise to the reaction temperature, and the reaction time was 40 seconds. The lactic acid concentration in the reaction solution prior to reaction was 1.085 M, and the aqueous ammonia concentration was 5.002 M. The aqueous solution obtained after the reaction was examined with a high performance liquid chromatography/ mass spectroscopy apparatus, whereby it was confirmed that 13.7 mM alanine had been produced. The reaction yield of alanine was 1.4%.

### Example 17

The continuous synthesis of alanine from lactic acid and liquefied ammonia was attempted by conducting the same reaction as in Example 1. A 0.556 M lactic acid aqueous solution was prepared using distilled water from which dissolved oxygen had been removed. Using the continuous reaction apparatus shown in Fig. 2, the 0.556 M lactic acid aqueous solution and the liquefied ammonia were injected into the reactor with two different water pumps.

Some of the reaction conditions, however, were changed as follows.

| Changed reaction conditions | |
|---|---|
| Reaction temperature | 380°C |
| Reaction pressure | 30 MPa |
| Density of high-temperature and high-pressure water | 0.5340 g/cm³ |
| Flow rate of carrier water | 10 mL/min |
| Flow rate of substrate solution (0.556 M lactic acid aqueous solution) | 0.5 mL/min |
| Flow rate of substrate solution (liquefied ammonium) | 1 mL/min |

The concentrations of the substrates prior to entering the reactor were 26.5 mM for lactic acid and 0.28 M for aqueous ammonia. The reaction time was 0.305 second, and the aqueous solution obtained after the reaction was examined with a high performance liquid chromatography/mass spectroscopy apparatus, whereby it was confirmed that an amino group had been introduced into the lactic acid, and that alanine had been produced. The alanine concentration was 5.4 mM, and the reaction yield thereof was 20.4%.

### INDUSTRTAL APPLICABILITY

As detailed above, the present invention relates to a method for introducing an amino group, wherein an amino group is introduced into an organic acid by reacting an organic acid with ammonia or an ammonium salt compound under high-temperature and high-pressure water conditions, and to a method for synthesizing an amino acid, wherein an organic acid and ammonia or an ammonium salt compound are reacted under high-temperature and high-pressure water conditions, and an amino acid is synthesized from the organic acid. The present invention affords the following effects: 1) A novel amino group introduction method that is conducted under high temperature and high pressure can be provided. 2) An amino acid can be synthesized by reacting an organic acid with ammonia or an ammonium salt compound under high temperature and high pressure. 3) An amino acid can be continuously synthesized at high speed from an organic acid by using the above-mentioned amino group introduction method being applied to a flow system. 4) An amino acid synthesis method that makes no use whatsoever of organic solvents or catalysts can be provided. 5) A high-purity amino acid can be manufactured. 6) The present invention is useful as a chemical substance production system that is safe for the environment.

## Claims

1. A method for introducing an amino group, which comprises reacting an organic acid with ammonia or an ammonium salt compound under high-temperature and high-pressure water conditions to introduce an amino group into the organic acid.

2. A method for synthesizing an amino acid, which comprises reacting an organic acid with ammonia or an ammonium salt compound under high-temperature and high-pressure water conditions, thereby introducing an amino group into the organic acid to synthesize an amino acid.

3. The method for synthesizing an amino acid according to Claim 2, wherein an organic acid is reacted with ammonia or an ammonium salt compound under high-temperature and high-pressure water conditions in which the temperature is at least 250°C and the pressure is at least pressure 20 MPa.

4. The method for synthesizing an amino acid according to Claim 2 or 3, wherein an organic acid is reacted with aqueous ammonia, ammonium acetate, or ammonium carbonate under high-temperature and high-pressure water conditions.

5. The method for synthesizing an amino acid according to Claim 2 or 3, wherein an organic acid is reacted with liquefied ammonia under high-temperature and high-pressure water conditions.

6. The method for synthesizing an amino acid according to one of Claims 2 to 5, wherein a hydroxy acid is used as the organic acid.

7. The method for synthesizing an amino acid according to one of Claims 2 to 6, wherein lactic acid, glycolic acid, malic acid, or tartaric acid is used as the organic acid.

8. The method for synthesizing an amino acid according to one of Claims 2 to 7, wherein an organic acid and ammonia or an ammonium salt compound are introduced into a reactor and are reacted continuously under high-temperature and high-pressure water conditions.

9. A method for manufacturing an amino acid using an organic acid and ammonia or an ammonium salt compound as the reaction substrates under high-temperature and high-pressure water conditions, which comprises introducing the organic acid and the ammonia or ammonium salt compound into a reactor, reacting them continuously under high-temperature and high-pressure water conditions, and then separating and refining the reaction solution thus obtained with an ion exchange resin or other such amino acid separation material to obtain an amino acid.
